# EUROPEAN PATENT SPECIFICATION

(11) **EP 3 157 485 B1**
(45) Date of publication and mention of the grant of the patent: **10.11.2021**
(21) Application number: 15809169.4
(22) Date of filing: 12.06.2015
(51) Int. Cl.: A61F 13/49, A61F 13/496, A61F 13/534

(54) **STRETCH BREATHABLE PROTECTIVE ABSORBENT ARTICLE USING TRI-LAMINATE**
ATMUNGSAKTIVER DEHNBARER SAUGFÄHIGER SCHUTZARTIKEL MIT TRILAMINAT
ARTICLE ABSORBANT PROTECTEUR RESPIRANT EXTENSIBLE UTILISANT UN STRATIFIÉ À TROIS COUCHES

(30) Priority: 17.06.2014 US 201414306386
(43) Date of publication of application: 26.04.2017
(73) Proprietor: Medline Industries, Inc., Mundelein, IL 60060 (US)
(72) Inventor: NELSON, Christopher, Plymouth, WI 53073 (US)
(74) Representative: Gaunt, Thomas Derrick
(86) International application number: PCT/US2015/035461
(87) International publication number: WO 2015/195467

(56) References cited:
- WO-A1-2015/164170
- WO-A1-2015/164173
- US-A- 4 916 005
- US-A- 5 034 008
- US-A- 5 295 988
- US-A1- 2007 239 131
- US-A1- 2008 287 899
- US-A1- 2010 163 161
- US-A1- 2010 168 705
- US-A1- 2011 144 610
- US-A1- 2014 130 956
- US-B2- 6 994 761

## Description

### FIELD OF THE INVENTION

The present invention relates to an absorbent article. More particularly, the invention relates to protective underwear that makes use of breathable laminate material having three layers.

### BACKGROUND OF THE INVENTION

Millions of people of all ages suffer from incontinence of the bowel or bladder. Whether an infant, adult, or elderly person, the underlying cause of incontinence varies but the method of treatment typically involves use of absorbent article products. Adult incontinent briefs, disposable diapers and underpads can alleviate some of the emotional and physical discomfort of incontinence by absorbing and containing liquid and other discharges from the human body to prevent body and clothing soiling.

A disadvantage of known disposable undergarments is that they are often constructed from materials that are designed to capture urine and other exudates and prevent leakage, but are not breathable. Consequently, moisture may become trapped between the

wearer and the disposable undergarment leading to discomfort and irritation. Further, as disposable undergarments are intended to replace traditional undergarments, disposable undergarments must be constructed to permit the wearer to be repeatedly put-on and pull-off the garment as necessary until such time as the garment is ready for disposal.

Disposable protective underwear products are known in the art. Such disposable underwear products rely on retractive forces that are provided by elastics, such as spandex strands. It is also known to use stretch elastic laminates that replace the spandex strands so as to provide better a fit to the wearer and improved discretion. Some products are created from a coextruded elastic layer made during the nonwoven manufacturing process to provide a product with improved breathability.

Widlund, et al., U.S. Patent No. 6,375,646 teaches a disposable diaper including an elongated absorbent pad, inner and outer casing layers and an elastically stretchable region in at least one of the front and back portions of the disposable diaper. The crotch portion of the disposable diaper is not stretchable. The combined stretchable and non-stretchable regions are designed to hold the absorbent material against the wearer's body to prevent leakage.

Norrby, et al., U.S. Patent No. 8,298,205 teaches an elastically stretchable laminate that includes a first non-elastic nonwoven web, a second non-elastic nonwoven web and an elastic film between the first and the second nonwoven webs. The laminate is rendered elastic in a first direction by incremental stretching and partial tearing of the first and second nonwoven webs.

Thorson, et al., U.S. Patent Application Publication No. 2011/0098668, teaches a disposable absorbent garment employing elastomeric film laminate body panels. The laminate can include an elastomeric film and nonwoven layers, and inner and outer surfaces adhered to nonwoven and elastomeric film layers.

Stablefeldt, et al., U.S. Patent Application Publication No. 2010/0168705, teaches disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Gilgenback U.S. Patent Application Publication No. 2010/0163161 teaches a process for making disposable absorbent garments employing elastomeric film laminates with deactivated regions. A portion of the disposable garment includes laminated elastomeric and non-elastomeric polymeric film layers and a nonwoven layer. An absorbent member extends partially through the laminated layers.

Kielpikowski, et al., U.S. Patent No. 4,842,596, teaches a method for making a breathable elastic fabric composite and personal article incorporating same. A liquid impermeable elastomeric film is sandwiched between pairs of nonwoven sheets. The elastomeric film is a partially stretched condition and bonded to the nonwoven sheets. The resulting laminated sheets create gathers that form breathable apertures.

Klemp, et al., U.S. Patent No. 6,994,761 teaches a disposable diaper and process for making the same. The diaper includes inner and outer portions that are ultrasonically bonded to create the vent sites or apertures through a layer of stretchable, breathable material.

Morrell-Schwartz, et al U.S. Patent Application Publication No. 2008/0287899 teaches a disposable diaper including an ultrasonically bonded stretchable film.

Nelson International Patent Application Publication No. WO2015/164170 teaches a disposable diaper including a bi-laminate that is bonded in a first zone and is glued in a second zone.

Nelson International Patent Application Publication No. WO2015/164173 teaches a disposable diaper including a tri-laminate that is bonded in a first region and is glued in a second region.

### SUMMARY OF THE INVENTION

### The invention is set out in the appended claims.

### BRIEF DESCRIPTION OF THE FIGURES

Advantages of the invention will become apparent upon reading the following detailed description and upon reference to the drawings.
FIG. 1 is a top plan view of laminate portions of an absorbent article in a substantially flat un-contracted position according to an arrangement not according to the invention.
FIG. 2 is a cross-sectional view of the laminate of FIG. 1 along sectional line A-A.
FIG. 3 is a cross-sectional view of a breathable laminate according to an arrangement not according to the invention.
FIG. 4 is a top view of the laminate of FIG. 3.
FIG. 5 is a top plan view of an arrangement not according to the invention similar to the absorbent article of FIG. 1 in a substantially flat un-contracted position and further including leg and waist elastics.
FIG. 6 is an alternative arrangement not according to the invention similar to the absorbent article of FIG. 5.
FIG. 7 is a top plan view ofan absorbent article according to an embodiment of the invention in a substantially flat un-contracted position and including a covering nonwoven layer.
FIG. 8 is a cross-sectional view of the absorbent article of FIG. 7 along sectional line B-B.
FIG. 9 is a top plan view of the embodiment of the absorbent article of FIG. 7 in a substantially flat un-contracted position and further showing the absorbent assembly.
FIG. 10 is an exploded perspective view of the absorbent article shown in FIG. 9.
FIG. 11 is a cross-sectional view of a breathable laminate for use in an embodiment of the invention.
FIG. 12 is a top view of the laminate of FIG. 11.

### DETAILED DESCRIPTION

Absorbent articles as described herein generally include a moisture-pervious inner layer, an absorbent layer, and a moisture-impervious outer layer. Although the remainder of the description will be specifically directed to adult incontinence articles, such as disposable diapers or briefs, it is to be understood that the embodiments may also be implemented using other absorbent articles and that the properties and uses described below apply to these other absorbent articles as well. Throughout this application, the terms absorbent article, diaper or brief are used interchangeably. However, it should be understood that the terms diaper or brief are intended to include other absorbent articles, such as training pants, incontinence pads, etc., as would be understood by one of ordinary skill in the art.

As used in the description herein and throughout the claims, the following terms take the meanings explicitly associated herein, unless the context clearly dictates otherwise: the meaning of "a," "an," and "the" includes plural reference, the meaning of "in" includes "in" and "on." Relational terms such as first and second, top and bottom, proximal and distal, and the like may be used solely to distinguish one entity or action from another entity or action without necessarily requiring or implying any actual such relationship or order between such entities or actions.

As used herein, the following terms have the following meanings:
"Attach" and its derivatives refer to the joining, adhering, connecting, bonding, sewing together, or the like, of two elements. Two elements will be considered to be attached together when they are integral with one another or attached directly to one another or indirectly to one another, such as when each is directly attached to intermediate elements. "Attach" and its derivatives include permanent, releasable, or refastenable attachment. In addition, the attachment can be completed either during the manufacturing process or by the end user.
"Bond" and its derivatives refer to the joining, adhering, connecting, attaching, sewing together, or the like, of two elements. Two elements will be considered to be bonded together when they are bonded directly to one another or indirectly to one another, such as when each is directly bonded to intermediate elements. "Bond" and its derivatives include permanent, releasable, or refastenable bonding.
"Connect" and its derivatives refer to the joining, adhering, bonding, attaching, sewing together, or the like, of two elements. Two elements will be considered to be connected together when they are connected directly to one another or indirectly to one another, such as when each is directly connected to intermediate elements. "Connect" and its derivatives include permanent, releasable, or refastenable connection. In addition, the connecting can be completed either during the manufacturing process or by the end user.
"Breathable" when used in describing a layer or multi-layer laminate means that the layer has the ability to allow moisture vapor to be transmitted through the material. Breathable layers may be air permeable, but it is not necessary to be air permeable to be breathable. In addition, breathable layers may be liquid permeable or liquid impermeable.
"Disposable" refers to articles that are designed to be discarded after a limited use rather than being laundered or otherwise restored for reuse.
The terms "disposed on," "disposed along," "disposed with," or "disposed toward" and variations thereof are intended to mean that one element can be integral with another element, or that one element can be a separate structure bonded to or placed with or placed near another element.
"Fiber" refers to a continuous or discontinuous member having a high ratio of length to diameter or width. Thus, a fiber may be a filament, a thread, a strand, a yarn, or any other member or combination of these members.
"Layer" when used in the singular can have the dual meaning of a single element or a plurality of elements.
"Liquid impermeable," when used in describing a layer or multi-layer laminate means that liquid, such as urine, will not pass through the layer or laminate, under ordinary use conditions, in a direction generally perpendicular to the plane of the layer or laminate at the point of liquid contact.
"Liquid permeable" refers to any material that is not liquid impermeable.
"Member" when used in the singular can have the dual meaning of a single element or a plurality of elements.
"Nonwoven" and "nonwoven web" refer to materials and webs of material that are formed without the aid of a textile weaving or knitting process. For example, nonwoven materials, fabrics or webs have been formed from many processes such as meltblowing processes, spunbonding processes, air laying processes, and bonded carded web processes.

These terms may be defined with additional language elsewhere in the specification.

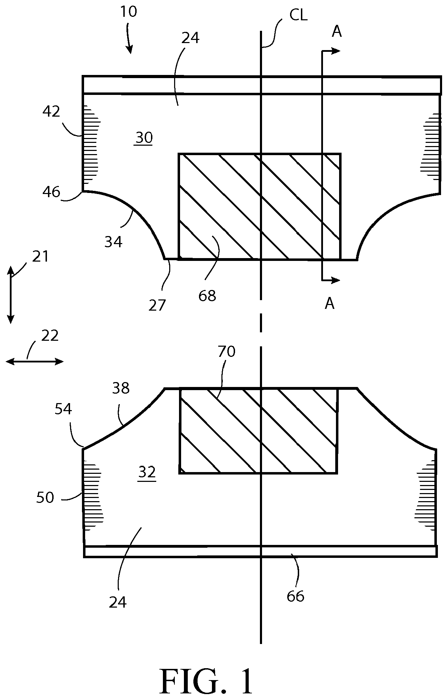

FIGS. 1, 5 and 6 illustrate plan views of an absorbent article 10 arrangement not according to the invention and are presented for illustration purposes only. In these figures the absorbent article 10 is shown in a substantially flat un-contracted state. As shown in these figures, the absorbent article 10 generally consists of several layers, including an inner layer, an absorbent layer, and an outer layer. The inner layer faces a wearer and contacts the skin of the wearer when the absorbent article 10 is secured to the wearer. The inner layer may comprise a topsheet that is composed of a moisture-pervious fabric suitable to allow bodily discharge to pass through the inner layer and be absorbed by the absorbent layer. Non-limiting examples of materials suitable to form the topsheet include polypropylene, polyethylene, polyester, materials having hydrophobic properties, combinations thereof and/or the like. Additionally, the topsheet can be treated with a hydrophilic finish to improve pass through of liquids to diaper layers beneath the inner layer. Non-limiting examples of suitable hydrophilic finishes include stearic acid, melamine-based chemicals, fluorocarbon chemicals, and silicon based chemicals.

The plan view of FIGS. 1, 5 and 6 is shown from the top or patient contacting side of the absorbent article. As illustrated in these figures, a particular arrangement of a disposable absorbent article 10 defines a longitudinal direction 21 parallel to a centerline CL and a transverse direction 22 perpendicular to the longitudinal direction. The absorbent article comprises a front section 12, a rear section 16, and a crotch section 14.

Referring to FIG. 1, the absorbent article includes a film layer 24 comprising a laminate film. The laminate film may be divided into two sections such that the film layer 24 forms at least part of the front section 12 and rear section 16. The front film section 30 is spaced apart from the rear film section 32 such that they are separated in the crotch section 14.

The front film section 30 defines a front end edge 26 and a front crotch edge 27 parallel to and longitudinally spaced from the front end edge 26. The rear film section 32 defines a rear end edge 28 longitudinally opposite the front end edge 26 and a rear crotch edge 29 parallel to and longitudinally spaced from the rear end edge 28. The front film section defines opposed front leg edges 34 and 36, and the rear film section defines opposed rear leg edges 36 and 38.

The front film section 30 further defines first and second transversely opposed front side edges 42 and 44. The first front side edge 42 extends in the longitudinal direction 21 from the front end edge 26 to a front intersection point 46 where the first front side edge intersects the first front leg edge 34. The second front side edge 44 extends in the longitudinal direction 21 from the front end edge 26 to a front intersection point 48 where the first front side edge intersects the second front leg edge 36. The rear film section 32 also defines first and second transversely opposed back side edges 50 and 52. The first back side edge 50 extends in the longitudinal direction 21 from the back end edge 28 to a rear intersection point 54 where the first rear side edge 50 intersects the first rear leg edge 38, and the second back side edge 52 extends in the longitudinal direction 21 from the back end edge 28 to a rear intersection point 56 where the second rear side edge 52 intersects the second rear leg edge 40.

The front section 30 is constructed at least in part of a laminate 24 that comprises a polymeric film layer 62 and at least two nonwoven layers 58,60, wherein both the polymeric film layer 62 and the nonwoven layers 58,60 extend substantially throughout the area of the laminate 24.

In its completed form as used by a wearer, the absorbent article includes a first side seam at which the first front side edge 42 is attached to the first back side edge 50 and which defines a first side seam length. The article further includes a second side seam at which the second front side edge 44 is attached to the second back side edge 52 and which defines a second side seam length. The article is accordingly formed into a brief or pull-up style disposable absorbent article.

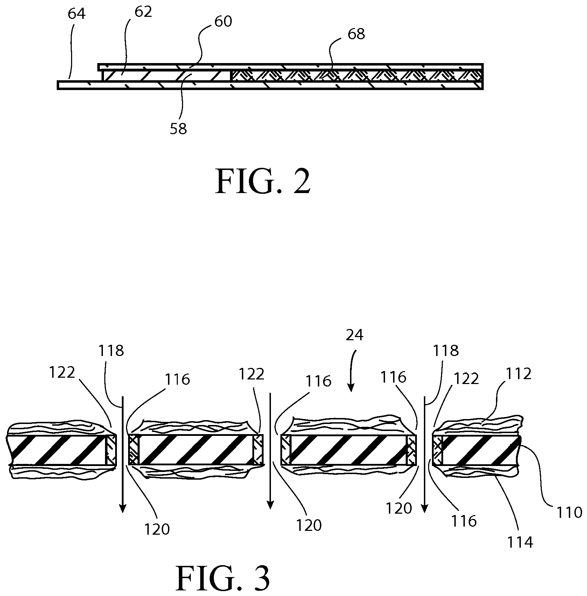

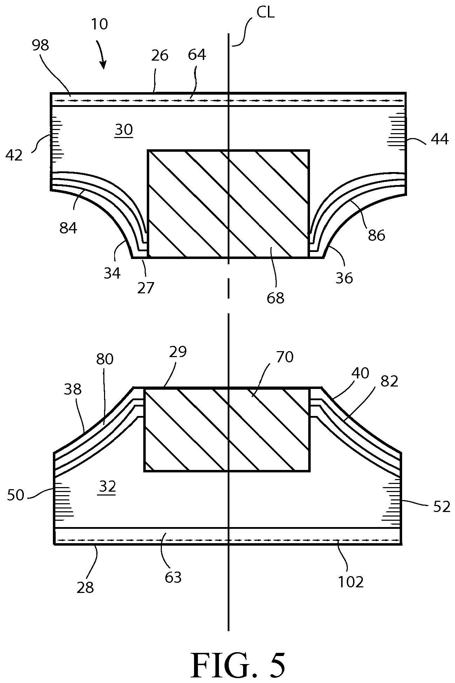

FIG. 2 shows a cross-sectional view of the laminate 24 along line A-A. The laminate 24 comprises two nonwoven layers 58, 60 superposed on opposing bottom and top surfaces of the polymeric film 62 such that the polymeric film 62 is sandwiched between the two nonwoven facings 58, 60, and both the polymeric film 62 and both nonwoven layers 58, 60 extend substantially through the area of the laminate 24. The polymeric film layer may be a block copolymer. A portion 64 of the bottom or outer layer of nonwoven 58 may extend beyond the polymeric film 62 along front end edge 26. Similarly (as illustrated among Figs. 5-6 and 9-10), a further portion 63 of the bottom or outer layer of nonwoven 58 may extend beyond the polymeric film 62 along rear end edge 28.

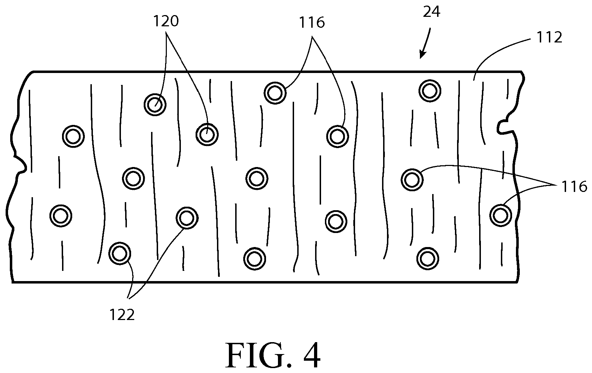

FIGS. 3-4 shows an arrangement not according to the invention and is presented for illustration purposes only. In this illustrative arrangement, the laminate 24 may be formed of a breathable cloth-like elastic nonwoven laminar fabric by sandwiching a liquid impermeable and non-self-adhering elastomeric film or nonwoven carrier sheet 110 between at least a pair of nonwoven facing sheets 112, 114 and bonding the facing sheets 112, 114 together by autogenous bonds, such as ultrasonically or thermally-generated bonds, through the carrier sheet 110 at spaced apart sites 116, thereby forming breathable apertures 120 through the carrier sheet which laminate the carrier and facing sheets together at the spaced apart sites 116.

In arrangements not according to the invention, the facing sheets 112, 114 and the elastomeric film 110 are ultrasonically bonded at sites 116. The ultrasonic bonding process creates a bond region 122 where the material from the top sheet 112 and the bottom sheet 114 mix together to form a bond. The bond region may also include mixing of the elastomeric film 110 material such that all three layers are bonded together. The ultrasonic bonding process may be configured such that it generates a through passage 120 generally within the confines of the bond region 122 in order to provide for the passage of water vapor 118 and give breathability to the laminate 24.

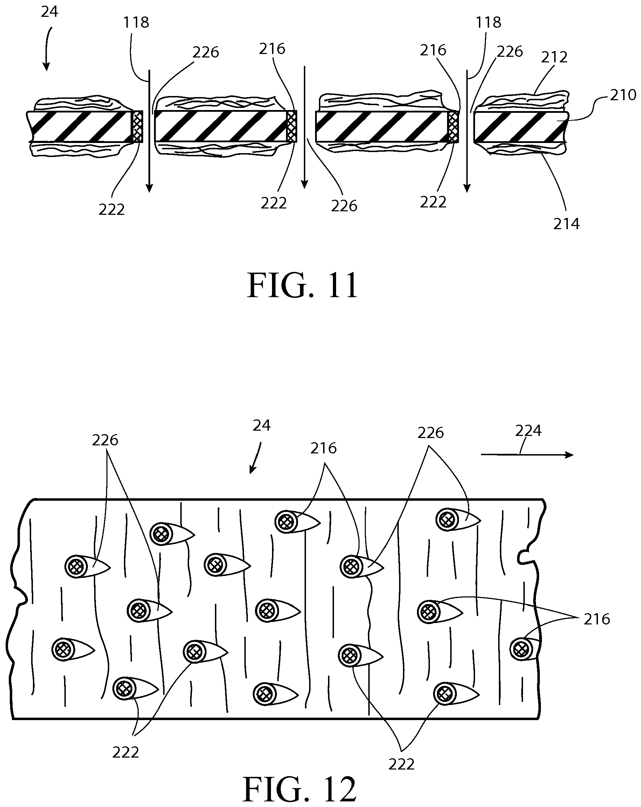

In embodiments of the invention, as illustrated in FIGS. 11-12, the facing sheets 212, 214 and the elastomeric film 210 are ultrasonically bonded at sites 216. The ultrasonic bonding process creates a bond region 222 where the material from the top sheet 212 and the bottom sheet 214 mix together to form a bond. The bond region 222 may also include mixing of the elastomeric film material 210 such that all three layers are bonded together and may form an impermeable layer within a perimeter of the bond site. The ultrasonic bonding process is conducted while the sheet is moving in a direction 224 such that a trailing tear 226 forms in the laminate generally outside and adjacent to the bond region 222. These trailing tears 226 provide through openings for the passage of water vapor 118 and give breathability to the laminate 24. While the bond regions 222 and trailing tears 226 are shown in FIGS. 11-12 as being of such a size and shape sufficiently large to illustrate the structure of the bonded laminate, one of skill in the art will understand that these may be of any appropriate size and shape and may be sufficiently small that they are not be readily apparent without the use of magnification.

In alternative arrangements not according to the invention, the laminate 24 can also be constructed such that the web is not made breathable during the ultrasonic laminating process, but rather has breathability imparted through a needling, slitting or die treatment process after formation of the complete laminate.

Referring again to FIG. 1, at least a portion 68 of the front film section 30 is non-elastomeric, and at least a portion 70 of the rear film section 32 is non-elastomeric. In FIGS. 1, 5-7 and 9 non-elastomeric or partially elastomeric regions are indicated by a pattern of hash lines, which lines are continuous if the non-elastomeric regions are exposed, and which lines are dashed if the non-elastomeric regions are concealed by an overlying component. In one preferred approach, as shall be described in more detail below, the entire laminate 24 is constructed of an elastomeric film laminate which includes an elastomeric film layer and at least two nonwoven facing layers, and a portion of the laminate has been "deactivated" or "deadened" to render it non-elastomeric.

As used herein, "elastomeric" refers to a material or composite which can be elongated by at least 50 percent of its relaxed length and which will recover, upon release of the applied force, at least 50 percent of its elongation. It is generally preferred that the elastomeric material or composite be capable of being elongated by at least 100 percent, more preferably by at least 200 percent, of its relaxed length and recover, upon release of an applied force, at least 50 percent of its elongation. "Non-elastomeric" refers to a material or composite that is non-extensible, or that is extensible but will recover no more than 20 percent of its elongated length after release of an applied elongating force. "Non-extensible" refers to a material that cannot stretch or extend by more than 25 percent of its relaxed length without fracture upon application of a biasing force. "Partially elastomeric" refers to a material or composite which can be elongated by at least 50 percent of its relaxed length and which will recover, upon release of the applied force, more than 20 percent but less than 50 percent of its elongation.

"Deactivated" as used herein to describe a material, region of a material, or regions of a material means that the material, region, or regions of material has been treated in some way to substantially destroy the elastic properties of the material, region, or regions, rendering the material, region, or regions non-elastomeric.

Deactivation of the non-elastic portions 68, 70 may be accomplished by a deactivation unit to create deactivated regions in the elastomeric film laminate 24. The deactivation can be accomplished by any of a variety of means. Frequently, some form of energy is applied to deactivate the non-elastic regions 68, 70, such as pressure, heat, ultrasonic energy, combinations thereof, and the like. Techniques employing pressure, heat, and ultrasonic energy are known in the art. The deactivation can occur in a variety of patterns. For example, the deactivating energy could be applied in a solid pattern, a series of vertical stripes, horizontal stripes, or diagonal stripes, a series of squares or dots, or other suitable pattern.

In the arrangement not according to the invention illustrated in FIG. 5, the absorbent article 10 comprises a first rear leg elastic member 80 attached to an inside surface of the rear film section 32 adjacent at least a portion of the first rear leg edge 38, and a second back leg elastic member 82 to an inside surface of the rear film section 32 adjacent at least a portion of the second rear leg edge 40. In further arrangements , the absorbent article 10 comprises a first front leg elastic member 84 attached to attached to an inside surface of the rear film section 30 adjacent at least a portion of the first front leg edge 34, and a second front leg elastic member 86 attached to attached to an inside surface of the rear film section 30 adjacent at least a portion of the second front leg edge 36. Each leg elastic member 80, 82, 84, 86 can comprise a single strand, ribbon, or strip of elastomeric material, or each can comprise two or more strands, ribbons, or strips, such as, for example, three strands (as depicted in FIG. 5). The leg elastic members 80, 82, 84, 86 may be glued in place or otherwise adhered to a top surface of nonwoven layer 60.

As illustrated in FIG. 5, rear leg elastic member 80, 82 extends from side edges 50, 52 of the rear film section 32 along rear leg edges 38, 40 to side edges of the non-elastic portion 70 of the rear film section. Alternatively, the rear leg elastic member 80, 82 may extend across part or the entire non-elastic portion 70. Likewise, front leg elastic member 84, 86 may extend from side edges 42, 44 of the front film section 30 along front leg edges 34, 36 to side edges of the non-elastic portion 68 of the front film section. Alternatively, the front leg elastic member 84, 86 may extend across part or the entire non-elastic portion 68.

For example, the first rear leg elastic member 80 and the second rear leg elastic member 82 may form part of a single, integral back elastic member that extends from the first rear side edge 50 transversely over the non-elastic portion 70 to the second rear side edge 52. Similarly, in arrangements the first front leg elastic member 84 and the second front leg elastic member 86 form part of a single, integral front elastic member that extends from the first front side edge 42 transversely over the non-elastic portion 68 to the second front side edge 44.

In arrangements , as illustrated in FIG. 5, the extension 64 of the outer nonwoven layer 58 (see FIGS. 1-2) of the front portion 30 may be folded over the top of inner nonwoven layer 60 to define the front end edge 26. A similar extension 63 of the outer layer of nonwoven of the rear laminate 32 may be folded over the top of inner nonwoven layer to define the rear end edge 28.

The absorbent article 10 further includes a front waist elastic member 98 positioned within the front fold 64 and a back waist elastic member 102 positioned within the back fold 63. In alternative arrangements, no front waist fold or back waist fold is included; instead opposite end edges of the laminate sections 30, 32 would define the front end edge 26 and back end edge 28, respectively. Each waist elastic member 98, 102 may comprise a single strand, ribbon, or strip of elastomeric material, or each can comprise two or more strands, ribbons, or strips.

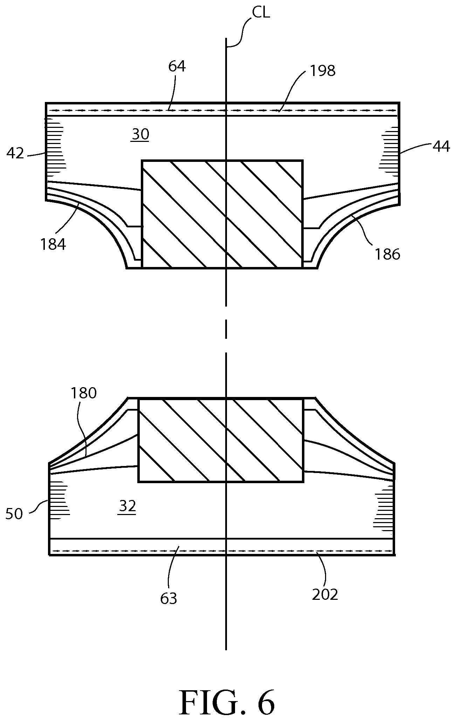

FIG. 6 illustrates a further arrangement not according to the invention in which the leg elastic members 180, 182, 184, 186 comprises a series of elastic strands. The illustrated arrangement shows three such strands, but more or fewer strands may be used. The leg elastics may be applied in a curved fashion. At the side edges 42, 44, 50, 52 of the diaper, the leg elastics are generally parallel, and each of the independent leg elastics are then curved towards the respective non-elastic portions 68, 70 of the film sections 30, 32, and increasingly separated in distance from one another the closer the leg elastics get to the non-elastic film portions. Also as shown in FIG. 6, the waist elastic members 198, 202 comprises multiple elastic strands.

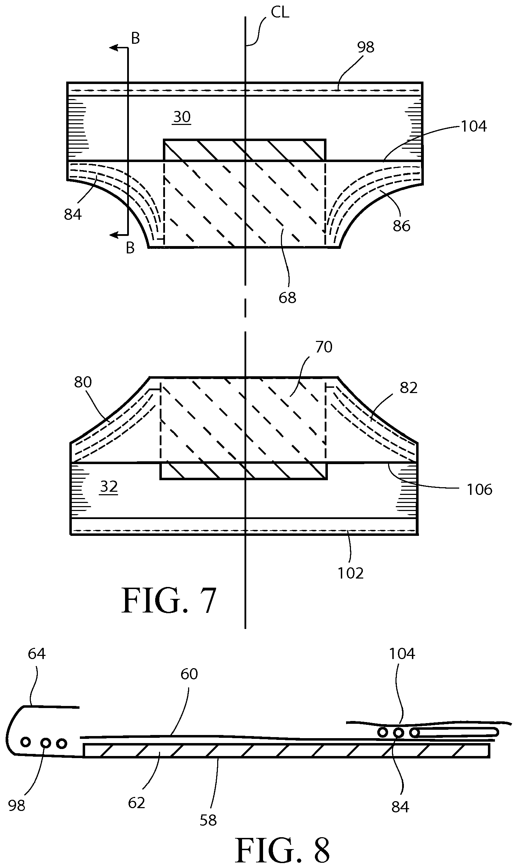

FIGS. 7-8 show an embodiment of the invention which is similar to the arrangement shown in Figure 1 but includes an additional covering nonwoven layer 104 attached to a top surface of the nonwoven layer 60 that comprises the top layer of the front laminate section 30. Additionally, a further covering nonwoven layer 106 may be attached to a top surface of the nonwoven layer 60 that comprises the top layer of the rear laminate section 32. The covering nonwoven layers 104, 106 are placed on top of laminate sections so that they at least in part cover the leg elastic elements. The covering nonwoven layers are adhesively bonded to the film laminate 24 in the region where the leg elastics 80, 82, 84, 86 are not present and may be glued to the elastics and/or the top laminate nonwoven layer 60 in regions where the elastics are located.

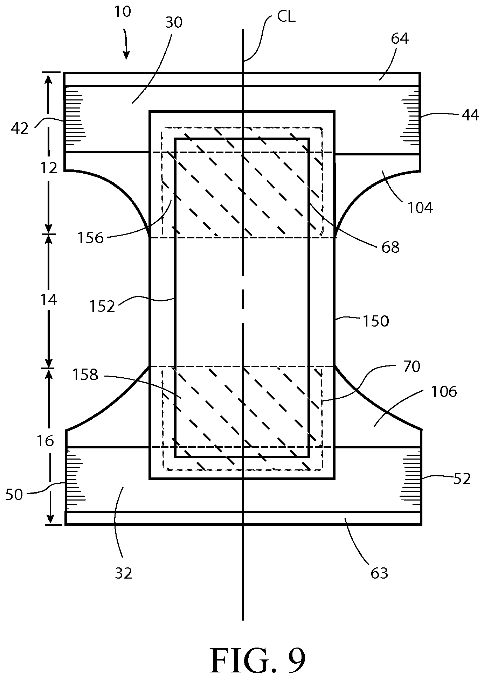

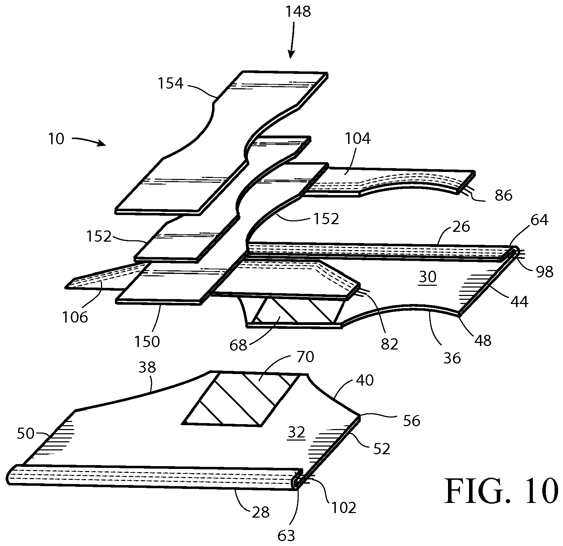

As illustrated in FIGS. 9-10, the absorbent article 10 of the illustrative embodiment also includes an absorbent assembly 148 that extends from the front section 12, across the crotch section 14, to the rear section 16. The absorbent assembly includes an absorbent core 152, and includes a topsheet 154 and a backsheet 150. (The topsheet 154 has been removed in FIG. 9 to more clearly show the position of the backsheet 150 and absorbent core 152.) The absorbent assembly 148 may be generally rectangular as shown in FIG. 9 or may comprise curved sections 166 to accommodate the wearer's legs as shown in FIG. 10. The absorbent core 152 may have an area that is smaller than the topsheet 154 and backsheet 150 such that the absorbent core is contained within the periphery of the absorbent assembly. The topsheet 154 and backsheet 150 may be bonded or otherwise adhered around a periphery of the absorbent assembly in order to capture the absorbent core 152 between the two sheets.

As shown in FIG. 9, the absorbent assembly 148 overlaps with the front section 30 to form a front overlapping zone 156, and the absorbent assembly 148 overlaps with the rear section 32 to form a rear overlapping zone 158. The periphery of the absorbent core 152 may be positioned completely within the front non-elastic portion 68 where the core overlaps with the front film section 30 and positioned completely within the rear non-elastic portion 70 where the core overlaps with the front film section 32. The backsheet 150 and topsheet (not shown) may also be positioned within the non-elastic portions 68, 70 in the overlapping zones, or may extend beyond the non-elastic portions as illustrated in FIG. 9.

In embodiments of the invention, the non-elastic portions 68, 70 may encompass more than 50% of the respective overlapping zones 156, 158. In other embodiments, more than 75%, and in further embodiments more than 90% of the area of the overlapping zones 156, 158 are non-elastomeric. In further embodiments, the entire of the overlapping zones are non-elastomeric. By adjusting the size of the non-elastic portions 68, 70 relative to the size of the absorbent core 152, the fit range of the article may be adjusted or the shape of the absorbent assembly may be defined in order to more readily capture exudate or prevent leaks.

In further embodiments, the non-elastic portions 68, 70 extend beyond the periphery of the absorbent core 152 or even beyond the backsheet 150. For example, non-elastic regions may be at least 10% larger, 20% larger, or in further embodiments 25% larger in area than the respective overlapped regions 156, 158. Providing non-elastic portions that are larger than their respective overlapping zones allows the process to accommodate any registration variability that may be present in the manufacturing process.

While the present invention has been described with reference to one or more particular embodiments, those skilled in the art will recognize that many changes may be made thereto without departing from the scope of the claims.

## Claims

1. An absorbent article, comprising:
a front laminate section (30) defining front end edge (26), a front crotch edge (27) parallel to and longitudinally spaced from the front end edge (26), a first and second leg edges (34, 36) and first and second transversely opposed side edges (42, 44) extending in a longitudinal directions, the front laminate section (30) comprising:
a front non-elastic portion (68) adjacent to the front crotch edge (27),
a front elastic portion (98) adjacent to the front end edge (26),
a polymeric film being an elastomeric film (62, 210),
a first nonwoven layer (60, 212),
a second nonwoven layer (58, 214),
a rear laminate section (32) defining rear end edge (28), a rear crotch edge (29) parallel to and longitudinally spaced from the rear end edge (28), and first and second transversely opposed side edges (50, 52) extending in a longitudinal directions, the rear laminate section (32) comprising a rear non-elastic portion (70) adjacent to the rear crotch edge (29) and a rear elastic portion (102) adjacent to the rear end edge (28);
an absorbent assembly (150, 152, 154) extending longitudinally between the front crotch edge (27) and the rear crotch edge (29), the absorbent assembly (150, 152, 154) comprising a topsheet (154), a back sheet (150) and an absorbent core (152) positioned between the topsheet (154) and backsheet (150); and
**characterized in that** the front laminate section (30) further comprises a plurality of spaced apart bonding sites (216) attaching the first nonwoven layer (60) to a first side of the polymeric film (62, 210) and the second nonwoven layer (58) to a second side of the polymeric film (62, 210) opposite the first nonwoven layer (60), and
a plurality of trailing tears (226) in the polymeric film (62, 210) outside and adjacent to the respective plurality of ultrasonic bonding sites (216), each trailing tear (226) forming a through passage that provides for the passage of water vapor (118) through the front laminate (30), wherein each of the plurality of trailing tears (226) is formed by movement of the polymeric film (62, 210) relative to the bonding site (216) during a bonding process; and
further **characterized in that** the absorbent article comprises a covering nonwoven layer (104) attached to a top surface of the first nonwoven layer (60), wherein the covering nonwoven layer (104) is adhesively bonded to the top surface of the first nonwoven layer (60) in a region where leg elastics (80, 82, 84, 86) are not present and is glued to the top surface of the first nonwoven layer (60) in a region where the elastics are present.

2. The absorbent article of claim 1 wherein the absorbent assembly (150, 152, 154) overlaps with the front non-elastic portion (68) and the rear non-elastic portion (70) and does not overlap with the front elastic portion (98) and the rear elastic portion (102).

3. The absorbent article of claim 1 wherein the front laminate section (30) further defines a first leg edge (34), the absorbent article further comprising a leg elastic (86) that is adhered to a surface of the front laminate section (30) and is parallel and adjacent to the leg edge (34) for at least a portion of the length of the leg elastic (86).

4. The absorbent article of claim 1 wherein the polymeric film (62) is a block copolymer.

5. The absorbent article of claim 1 wherein the non-elastic portion (68) of the front laminate (30) is formed by deactivating a portion of the elastomeric film (62, 210).

6. The absorbent article of claim 1 wherein the bonding sites (216) are ultrasonic bonds.

7. The absorbent article of claim 6 wherein the ultrasonic bonding sites (216) comprise a mixture of material from the first nonwoven layer (212), the polymeric film (210) and the second nonwoven layer (214) such that the first nonwoven layer (212), the polymeric film (210) and the second nonwoven layer (214) are bonded together (222).

8. The absorbent article of claim 7 wherein each ultrasonic bonding site (216) forms an impermeable layer within a perimeter of the bonding site (216).

9. The absorbent article of claim 1 further comprising a second plurality of through passages comprising perforations formed by passing a perforating tool through the front laminate.

## Patentansprüche

1. Ein absorbierender Artikel, umfassend:
Einen vorderen Schichtstoffabschnitt (30), der einen vorderen End-Rand (26), einen vorderen Schritt-Rand (27) parallel und längs, mit Zwischenraum versehen, zu dem vorderen End-Rand (26), einen ersten und zweiten Bein-Rand (34, 36) und einen ersten und zweiten quer gegenüberliegenden Seiten-Rand (42, 44), die sich in Längsrichtung erstrecken, definiert, der vordere Schichtstoffabschnitt (30) umfassend:
Einen vorderen nichtelastischen Abschnitt (68) anliegend am vorderen Schritt-Rand (27),
einen vorderen elastischen Abschnitt (98) anliegend am vorderen End-Rand (26),
eine Polymerfolie, die eine Elastomerfolie (62, 210) ist,
eine erste Vliesschicht (60, 212),
eine zweite Vliesschicht (58, 214),
einen hinteren Schichtstoffabschnitt (32), der einen hinteren End-Rand (28) definiert, einen hinteren Schritt-Rand (29) parallel und längs, mit Zwischenraum versehen, zu dem hinteren End-Rand (28), einen ersten und zweiten quer gegenüberliegenden Seiten-Rand (50, 52), die sich in Längsrichtung erstrecken, der hintere Schichtstoffabschnitt (32) umfasst einen hinteren nichtelastischen Abschnitt (70) anliegend am hinteren Schritt-Rand (29) und einen hinteren elastischen Abschnitt (102) anliegend am hinteren End-Rand (28);
eine absorbierende Anordnung (150, 152, 154), die sich längs zwischen dem vorderen Schritt-Rand (27) und dem hinteren Schritt-Rand (29) erstreckt, die absorbierende Anordnung (150, 152, 154) umfasst eine Deckschicht (154), eine untere Lage (150) und eine absorbierende Kernschicht (152), die zwischen der Deckschicht (154) und der unteren Lage (150) positioniert ist; und
**gekennzeichnet dadurch, dass** der vordere Schichtstoffabschnitt (30) ferner eine Vielzahl mit Zwischenräumen versehene Bindungsstellen (216) umfasst, die die erste Vliesschicht (60) mit einer ersten Seite der Polymerfolie (62, 210) und die zweite Vliesschicht (58) mit einer zweiten Seite der Polymerfolie (62, 210) verbinden, die der ersten Vliesschicht (60) gegenüberliegt, und
eine Vielzahl von Schlitzen (226) in der Polymerfolie (62, 210) außerhalb und anliegend an der entsprechenden Vielzahl von Ultraschall-Bindungsstellen (216), jeder Schlitz (226) bildet einen Durchgang, der Wasserdampf (118) durch den vorderen Schichtstoff (30) hindurchlässt, wobei jeder der Vielzahl von Schlitzen (226) durch die Bewegung der Polymerfolie (62, 210) im Verhältnis zu der Bindungsstelle (216) während eines Bindungsvorgangs gebildet wird; und
ferner **gekennzeichnet dadurch, dass** der absorbierende Artikel eine abdeckende Vliesschicht (104) umfasst, die an einer Oberfläche der ersten Vliesschicht (60) befestigt ist,
wobei die abdeckende Vliesschicht (104) haftend mit der Oberfläche der ersten Vliesschicht (60) in einem Bereich verbunden ist, in dem keine Bein-Gummizüge (80, 82, 84, 86) vorhanden sind, und mit der Oberfläche der ersten Vliesschicht (60) in einem Bereich verklebt ist, in dem die Gummizüge vorhanden sind.

2. Der absorbierende Artikel nach Anspruch 1, wobei die absorbierende Anordnung (150, 152, 154) den vorderen nichtelastischen Abschnitt (68) und den hinteren nichtelastischen Abschnitt (70) überlappt, aber den vorderen elastischen Abschnitt (98) und den hinteren elastischen Abschnitt (102) nicht überlappt.

3. Der absorbierende Artikel nach Anspruch 1, wobei der vordere Schichtstoffabschnitt (30) ferner einen ersten Bein-Rand (34) definiert, und der absorbierende Artikel ferner einen Bein-Gummizug (86) umfasst, der an einer Oberfläche des vorderen Schichtstoffabschnitts (30) befestigt ist und parallel und anliegend am ersten Bein-Rand (34) mindestens an einem Teil der Länge des Bein-Gummizugs (86) verläuft.

4. Der absorbierende Artikel nach Anspruch 1 wobei die Polymerfolie (62) ein Blockpolymer ist.

5. Der absorbierende Artikel nach Anspruch 1, wobei der nichtelastische Abschnitt (68) des vorderen Schichtstoffs (30) durch die Deaktivierung eines Abschnitts der Elastomerfolie (62, 210) gebildet wird.

6. Der absorbierende Artikel nach Anspruch 1, wobei die Bindungsstellen (216) Ultraschall-Bindungen sind.

7. Der absorbierende Artikel nach Anspruch 6 wobei die Ultraschall-Bindungsstellen (216) eine Mischung aus Material aus der ersten Vliesschicht (212), der Polymerfolie (210) und der zweiten Vliesschicht (214) umfasst, so dass die erste Vliesschicht (212), die Polymerfolie (210) und die zweite Vliesschicht (214) miteinander verbunden sind (222).

8. Der absorbierende Artikel nach Anspruch 7 wobei jede Ultraschall-Bindungsstelle (216) innerhalb des Umfangs der Bindungsstelle (216) eine undurchlässige Schicht bildet.

9. Der absorbierende Artikel nach Anspruch 1 ferner umfassend eine zweite Vielzahl von Durchgangsöffnungen, die Perforierungen umfasst, die dadurch geformt wurden, dass ein Perforationswerkzeug durch den vorderen Schichtstoff geführt wurde.

## Revendications

1. Article absorbant, comprenant :
une section de stratifié avant (30) définissant le bord d'extrémité avant (26), un bord d'entrejambe avant (27) parallèle et espacé longitudinalement du bord d'extrémité avant (26), un premier et second bords de jambe (34, 36) et les premier et second bords latéraux opposés de manière transversale (42, 44) s'étendant dans une direction longitudinale, la section de stratifié avant (30) comprenant :
une partie non élastique avant (68) adjacente au bord d'entrejambe avant (27),
une partie élastique avant (98) adjacente au bord d'extrémité avant (26),
un film polymère étant un film élastomère (62, 210),
une première couche non tissée (60, 212),
une seconde couche non tissée (58, 214),
une section de stratifié arrière (32) définissant le bord d'extrémité arrière (28), un bord d'entrejambe arrière (29) parallèle et espacé longitudinalement à partir du bord d'extrémité arrière (28) et les premier et second bords latéraux opposés de manière transversale (50, 52) s'étendant dans des directions longitudinales, la section de stratifié arrière (32) comprenant une partie non élastique arrière (70) adjacente au bord d'entrejambe arrière (29) et une partie élastique arrière (102) adjacente au bord d'extrémité arrière (28) ;
un ensemble absorbant (150, 152, 154) s'étendant longitudinalement entre le bord d'entrejambe avant (27) et le bord d'entrejambe arrière (29), l'ensemble absorbant (150, 152, 154) comprenant une feuille supérieure (154), une feuille inférieure (150) et un cœur absorbant (152) positionné entre la feuille supérieure (154) et la feuille inférieure (150) ; et
**caractérisé en ce que** la section de stratifié avant (30) comprend en outre une pluralité de sites de liaison espacés (216) fixant la première couche non tissée (60) à un premier côté du film polymère (62, 210) et la seconde couche non tissée (58) à un second côté du film polymère (62, 210) en regard de la première couche non tissée (60), et
une pluralité de déchirures de queue (226) dans le film polymère (62, 210) à l'extérieur et adjacentes à la pluralité respective de sites de liaison ultrasonore (216), chaque déchirure de queue (226) formant un passage traversant qui permet le passage de la vapeur d'eau (118) à travers le stratifié avant (30), dans lequel chacune de la pluralité de déchirures de queue (226) est formée par le déplacement du film polymère (62, 210) par rapport au site de liaison (216) pendant un processus de liaison ; et
**caractérisé en outre en ce que** l'article absorbant comprend une couche non tissée de couverture (104) fixée à une surface supérieure de la première couche non tissée (60),
dans lequel la couche non tissée de couverture (104) est collée par adhérence à la surface supérieure de la première couche non tissée (60) dans une région où les élastiques de jambe (80, 82, 84, 86) ne sont pas présents et est collée à la surface supérieure de la première couche non tissée (60) dans une région où les élastiques sont présents.

2. Article absorbant selon la revendication 1 dans lequel l'ensemble absorbant (150, 152, 1 , 54) chevauche la partie non élastique avant (68) et la partie non élastique arrière (70) et ne chevauche pas la partie élastique avant (98) et la partie élastique arrière (102).

3. Article absorbant selon la revendication 1, dans lequel la section de stratifié avant (30) définit en outre un premier bord de jambe (34) l'article absorbant comprenant en outre un premier élastique de jambe (86) qui est collé à une surface de la section de stratifié avant (30) et est parallèle et adjacent au bord de jambe (34) pour au moins une partie de la longueur de l'élastique de jambe (86).

4. Article absorbant selon la revendication 1 dans lequel le film polymère (62) est un copolymère à blocs.

5. Article absorbant selon la revendication 1 dans lequel la partie non élastique (68) du stratifié avant (30) est formée par la désactivation d'une partie du film élastomère (62, 210).

6. Article absorbant selon la revendication 1 dans lequel les sites de liaison (216) sont des liaisons ultrasonores.

7. Article absorbant selon la revendication 6 dans lequel les sites de liaison ultrasonore (216) comprennent un mélange de matériau provenant de la première couche non tissée (212), du film polymère (210) et de la seconde couche non tissée (214) de sorte que la première couche non tissée (212), le film polymère (210) et la seconde couche non tissée (214) sont collés ensemble (222).

8. Article absorbant selon la revendication 7 dans lequel chaque site de liaison ultrasonore (216) forme une couche imperméable à l'intérieur d'un périmètre d'un site de liaison (216).

9. Article absorbant selon la revendication 1, comprenant en outre une seconde pluralité de passages traversants comprenant des perforations formées par le passage d'un outil perforant à travers le stratifié avant.
